# EUROPEAN PATENT SPECIFICATION

(11) **EP 2 090 228 B1**
(45) Date of publication and mention of the grant of the patent: **13.10.2010**
(21) Application number: 06834722.8
(22) Date of filing: 14.12.2006
(51) Int. Cl.: A61B 6/03

(54) **DISPLAY DIRECTION CORRECTING DEVICE, CORRECTING METHOD, AND CORRECTION PROGRAM FOR MEDICAL 3D IMAGE**
VORRICHTUNG ZUR KORREKTUR DER ANZEIGERICHTUNG, KORREKTURVERFAHREN UND KORREKTURPROGRAMM FÜR EIN MEDIZINISCHES 3D-BILD
DISPOSITIF DE CORRECTION DE LA DIRECTION D'AFFICHAGE, PROCEDE DE CORRECTION ET PROGRAMME DE CORRECTION POUR IMAGE MEDICALE 3D

(43) Date of publication of application: 19.08.2009
(73) Proprietor: Imagnosis Inc., Kobe-shi Hyogo 658-0032 (JP)
(72) Inventor: KIM, Han-Joon, Kobe-shi Hyogo 658-0032 (JP)
(74) Representative: Steil, Christian
(86) International application number: PCT/JP2006/324968
(87) International publication number: WO 2008/072335

(56) References cited:
- EP-A- 1 207 495
- EP-A- 1 449 481
- WO-A1-01/03065
- FR-A- 2 850 775
- JP-A- 2002 360 564
- JP-A- 2002 360 564

## Description

### TECHNICAL FIELD

The present invention relates to display of a medical three-dimensional image and, particularly, to an apparatus, a method and a program for modifying the display orientation of a medical three-dimensional image.

### BACKGROUND ART

In the medical field, medical three-dimensional images such as CT imaging data are used for morphological evaluation and measurement of craniofacial bones. The morphological evaluation of the craniofacial bones is useful for surgical treatment to be performed to treat displaced or deformed maxillofacial bones. Further, evaluation of a distortion of several millimeters is significant for diagnosis and treatment.
Accordingly, definition of the orientation of the craniofacial bones is important for the evaluation and the measurement of the craniofacial bones. Therefore, the coordinate system which determines the orientation should be finely adjusted to permit accurate evaluation and measurement of the craniofacial bones.

A prior art technique for displaying a medical three-dimensional image is disclosed, in which anatomical characteristic points (hereinafter referred to as "landmarks") are extracted from the displayed three-dimensional image, and a reference coordinate system is determined in an anatomically unique manner based on the characteristic points. The three-dimensional image to be displayed is subjected to coordinate transformation based on the reference coordinate system, and the resulting medical three-dimensional image is displayed (see, for example, JP-A-HEI8(1996)-131403 of Patent Document 1).
Patent Document 1: JP-A-HEI8(1996)-131403
Patent Document 2: US Patent No. 6888546

### DISCLOSURE OF THE INVENTION

### PROBLEMS TO BE SOLVED BY THE INVENTION

However, the orientation of the displayed three-dimensional image varies depending on the positions of the landmarks employed for the determination of the reference coordinate system and the definition of image coordinates. That is, the reference coordinate system determined in the anatomically unique manner is not unique, but a plurality of reference coordinate systems are present. In the diagnostic imaging, therefore, a plurality of reference coordinate systems are properly defined under preconditions, and evaluation should be comprehensively made based on the reference coordinate systems.
In the prior art technique for displaying the medical three-dimensional image, when the reference coordinate system once determined is modified, landmarks different from the landmarks used for the determination of the reference coordinate system should be employed. Therefore, the original reference coordinate system is lost.

That is, the display orientation based on the reference coordinate system uniquely determined based on the landmarks is insufficient for displaying the medical three-dimensional image in a desired orientation and defining the coordinate system for clinical applications of the medical three-dimensional image. Hence, there is a demand for processes for flexibly setting the orientation and the coordinate system in a reproducible manner, but apparatuses, methods and programs for these processes have not been provided yet.
The present invention solves the problems associated with the prior-art medical three-dimensional image display method described above. It is therefore an object of the present invention to provide a medical three-dimensional image display orientation modifying apparatus, method and program which permit modification of the display orientation of an image based on a reference coordinate system once determined.

### MEANS FOR SOLVING THE PROBLEMS

The present invention solves the problems by providing an apparatus, a method and a program for newly defining a coordinate system having a unique and reproducible orientation based on a reference coordinate system determined based on a landmark and a displayed image by utilizing an additional landmark different from the landmark used for the determination of the reference coordinate system.
According to an aspect as set forth in claim 1, more specifically, there is provided a medical three-dimensional image display orientation modifying apparatus, which includes: displaying means that displays a medical three-dimensional image based on a three-dimensional coordinate system defined based on a reference landmark contained in the image; registering means that, when a pair of right and left landmarks different from the reference landmark used for the definition of the original three-dimensional coordinate system are specified in right and left regions of the displayed medical three-dimensional image, registers the specified landmarks; and modifying means that modifies inclinations of two of three axes of the three-dimensional coordinate system based on the pair of registered right and left landmarks with the other axis kept unchanged.

According to an inventive aspect as set forth in claim 2, the original medical three-dimensional image includes an image of a human head displayed based on a horizontal reference plane, a frontal plane and a median plane defined based on the reference landmark, and the modifying means modifies inclinations of the horizontal reference plane and the median plane based on the pair of registered right and left landmarks with the frontal plane kept unchanged in the medical three-dimensional image display orientation modifying apparatus of claim 1.

According to an inventive aspect as set forth in claim 3, the original medical three-dimensional image includes an image of a human head displayed based on a horizontal reference plane, a frontal plane and a median plane defined based on the reference landmark, and the modifying means modifies inclinations of the frontal plane and the median plane based on the pair of registered right and left landmarks with the horizontal reference plane kept unchanged in the medical three-dimensional image display orientation modifying apparatus of claim 1.

According to an inventive aspect as set forth in claim 4, a plurality of landmarks are specified for each of the right and left landmarks to be registered, and the right and left landmarks to be registered are each defined by a middle point of the plurality of landmarks in the medical three-dimensional image display orientation modifying apparatus of any of claims 1 to 3.
According to an inventive aspect as set forth in claim 5, there is provided a medical three-dimensional image display orientation modifying method, which includes the steps of: displaying a medical three-dimensional image based on a three-dimensional coordinate system defined based on a reference landmark contained in the image; when a pair of landmarks different from the reference landmark used for the definition of the original three-dimensional coordinate system are specified in the displayed medical three-dimensional image for modification of the coordinate system, registering the specified landmarks; and modifying inclinations of two of three orthogonal planes of the three-dimensional coordinate system based on the pair of registered landmarks with the other plane kept unchanged.

According to an inventive aspect as set forth in claim 6, there is provided a medical three-dimensional image display orientation modifying program which causes an apparatus as recited in any of claims 1 to 4 to perform a medical three-dimensional image display orientation modifying process.
While the apparatuses, the method and the program have been separately described as the means for solving the problems, the present invention is embodied with the use of a computer system. Therefore, the present invention is applicable to a computer system which is capable of performing the process sequence according to the present invention, a program prepared for performing the process according to the present invention, and a processing method including the process sequence.

### EFFECTS OF THE INVENTION

According to the present invention, when the medical three-dimensional image is displayed based on the three-dimensional coordinate system defined based on the reference landmark, the display orientation of the medical three-dimensional image can be modified based on the newly specified landmarks. The three-dimensional coordinate system is modified based on the reference coordinate system defined based on the reference landmark and the displayed image by utilizing the additional landmarks different from the reference landmark used for defining the original coordinate system. Thus, the display orientation and the coordinate system can be uniquely established with sufficient reproducibility.

For example, one of the orthogonal three axes of the three-dimensional coordinate system is kept unchanged, and the inclinations of the other two axes are modified by rotating the other two axes about the unchanged one axis.
Alternatively, one of the orthogonal three planes of the three-dimensional coordinate system is kept unchanged, and the inclinations of the other two planes are modified by rotating the other two planes about intersection lines between the unchanged one plane and the other two planes.

As a result, the modified three-dimensional coordinate system is based on the landmarks, so that the medical three-dimensional image is displayed based on the new coordinate system resulting from the modification based on the landmarks. Therefore, the medical three-dimensional image can be displayed in any of various orientations in a reproducible manner based on the landmarks.
According to the present invention, the display orientation of the three-dimensional image can be more flexibly set for the morphological evaluation and analysis of the three-dimensional image, so that the clinical applicability of the information of the three-dimensional image is significantly improved.

According to a preferred embodiment of the present invention, the inclination of the horizontal reference plane of the three-dimensional coordinate system is modified based on the newly registered landmarks.
With reference to the attached drawings, the present invention will hereinafter be described more specifically by way of embodiments thereof.

### BRIEF DESCRIPTION OF THE DRAWINGS

Fig. 1 is a block diagram showing the construction of a computer system which displays an image according to one embodiment of the present invention.
Fig. 2 is a schematic diagram showing an exemplary medical three-dimensional image displayed on a display device 2 shown in Fig. 1.
Fig. 3 is a schematic diagram showing another exemplary medical three-dimensional image displayed on the display device 2 shown in Fig. 1 , more specifically, showing a three-dimensional image of a human head displayed based on a horizontal reference plane HP, a frontal plane VP1 and a median plane VP2.
Fig. 4 is a diagram for explaining a method for modifying an X-axis in a three-dimensional image of the human head displayed based on landmark-based reference axes shown in Fig. 2.
Fig. 5 is a schematic diagram for explaining how to specify an additional landmark in an image of the human head as viewed from a left side of the human head.
Fig. 6 shows an exemplary three-dimensional image displayed as viewed perpendicularly to the median plane VP2 from a right side of the human head.
Fig. 7 is a diagram for explaining how to modify the horizontal reference plane HP and define the modified horizontal reference plane as a new horizontal reference plane.
Fig. 8 is a diagram for explaining how to modify the frontal plane VP1.
Fig. 9 is a flow chart showing a process sequence to be performed by a control section 1 for modifying reference axes.
Fig. 10 is a flow chart showing a process sequence to be performed by the control section 1 for modifying reference planes.
Fig. 11 is a schematic diagram for explaining a reference coordinate system modifying method according to another embodiment of the present invention.
Fig. 12 is a schematic diagram for explaining the reference coordinate system modifying method according to the another embodiment of the present invention.
Fig. 13 is a schematic diagram for explaining the reference coordinate system modifying method according to the another embodiment of the present invention.
Fig. 14 is a schematic diagram for explaining the reference coordinate system modifying method according to the another embodiment of the present invention.
Fig. 15 is a schematic diagram for explaining the reference coordinate system modifying method according to the another embodiment of the present invention.
Fig. 16 is a schematic diagram for explaining a reference coordinate system modifying method according to further another embodiment of the present invention.
Fig. 17 is a schematic diagram for explaining the reference coordinate system modifying method according to the further another embodiment of the present invention.
Fig. 18 is a schematic diagram for explaining the reference coordinate system modifying method according to the further another embodiment of the present invention.
Fig. 19 is a schematic diagram for explaining the reference coordinate system modifying method according to the further another embodiment of the present invention.

### DESCRIPTION OF REFERENCE CHARACTERS

- 1:: Control section
- 2:: Display device
- 3:: Keyboard
- 4:: Mouse
- 5:: Recording medium
- HP:: Horizontal reference plane
- VP1:: Frontal plane (first vertical reference plane)
- VP2:: Median plane (second vertical reference plan)

### BEST MODE FOR CARRYING OUT THE INVENTION

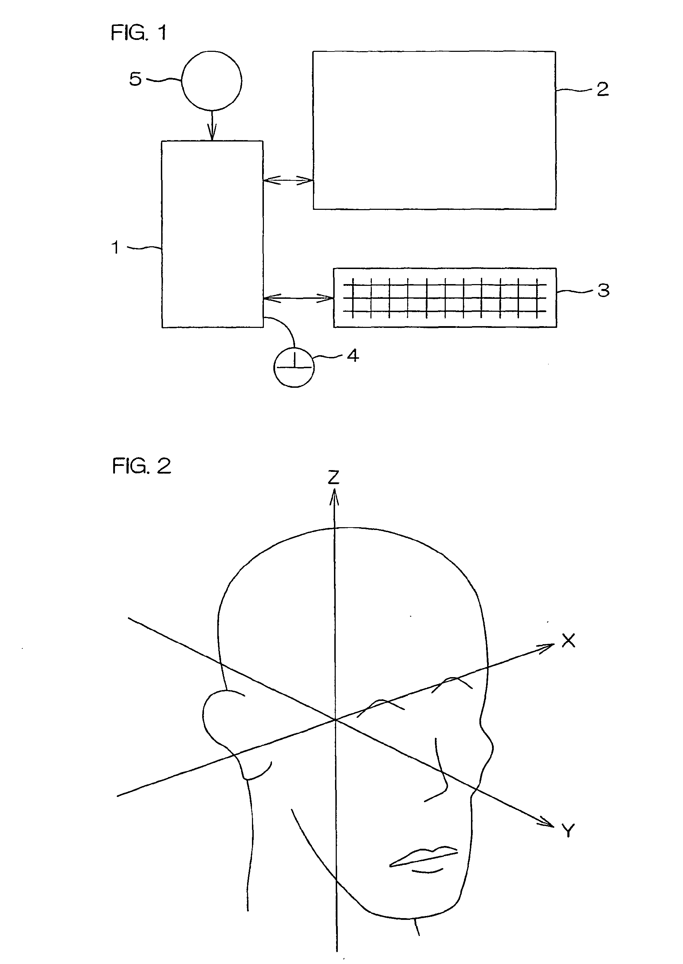

Fig. 1 is a block diagram showing the construction of a computer system adapted for image display according to one embodiment of the present invention. The computer system for the image display includes a control section 1 including a CPU, a ROM, a RAM and a hard disk, a display device 2 connected to the control section 1, a keyboard 3 and a mouse 4. The control section 1 is configured such that a program according to the embodiment of the present invention is installable from a recording medium 5.

Such a computer-based display system may be embodied by using an existing computer system.
Fig. 2 is a schematic diagram showing an exemplary medical three-dimensional image displayed on the display device 2 shown in Fig. 1. The medical three-dimensional image shown in Fig. 2 represents a human head, and reference axes, i.e. , an X-axis, a Y-axis and a Z-axis, which serve as a reference for the orientation and the coordinates of the three-dimensional image are also displayed.

The X-axis, the Y-axis and the Z-axis are reference axes which are defined based on anatomical characteristic points (hereinafter referred to as "landmarks") in the image. A method disclosed in JP-A-HEI8(1996)-131403, for example, may be employed for defining the reference axes based on the landmarks.
That is, the coordinate system is determined in conformity with the following rules: a middle point of a line segment connecting right and left ear holes is defined as an origin; a line parallel to a line extending through the centers of right and left eye balls is defined as the X-axis; a line perpendicular to the line extending through the centers of the right and left eye balls and the Z-axis is defined as the Y-axis; and a line perpendicular to a plane extending through the centers of the right and left eye balls and the origin is defined as the Z-axis.

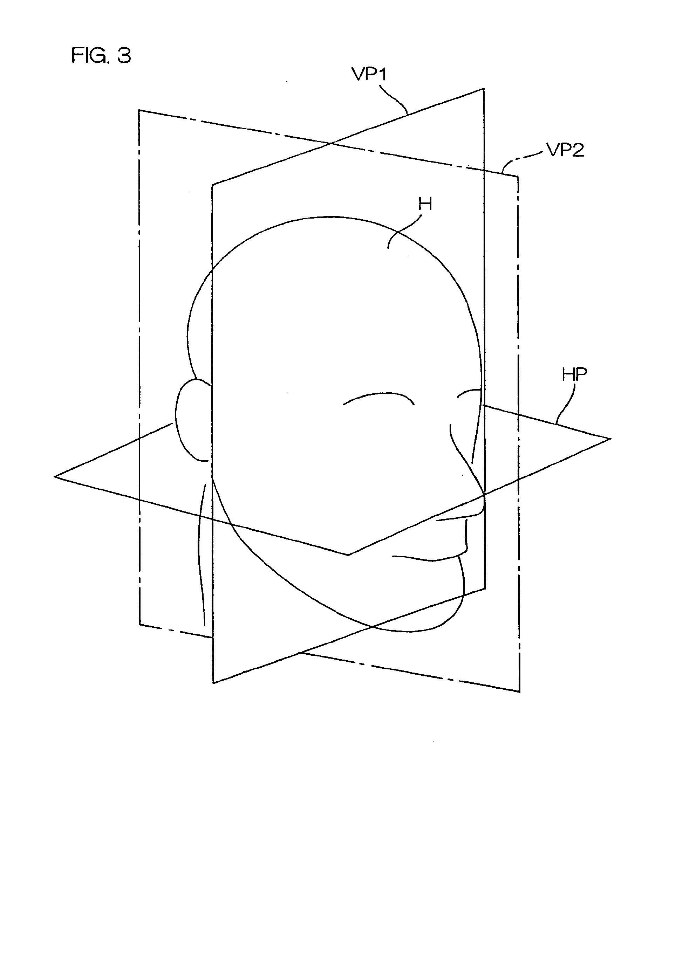

By thus defining the reference axes, i.e., the X-axis, the Y-axis and the Z-axis, based on the landmarks, a plurality of medical three-dimensional images displayed base on the reference axes can be orientated in the same direction for evaluation thereof. Therefore, the medical three-dimensional images can be accurately measured and evaluated. A basic medical three-dimensional image is not limited to that displayed based on the reference axes (the X-axis, the Y-axis and the Z-axis) defined based on the landmarks as shown in Fig. 2, but may be an image displayed based on reference planes as shown in Fig. 3.

A three-dimensional image of a human head shown in Fig. 3 is displayed based on a horizontal reference plane HP, a frontal plane (first vertical reference plane) VP1 and a median plane (second vertical reference plane) VP2. The horizontal reference plane HP, the frontal plane VP1 and the median plane VP2 are also defined based on the landmarks.
A method disclosed in US Patent No. 6888546 of Patent Document 2 is employed for defining the reference planes based on the landmarks.

The three-dimensional image shown in Fig. 2 and the three-dimensional image shown in Fig. 3 are displayed based on the reference axes or the reference planes defined based on the landmarks and, for morphological evaluation of facial bones in the three-dimensional image (of the human head), the three-dimensional image can be displayed in a predetermined orientation based on the landmarks with improved reproducibility.
If it is possible to define a new X-axis or a new horizontal reference plane based on the position of a landmark present neither on the X-axis nor on the Y-axis or the position of a landmark not present on the horizontal reference plane HP in the three-dimensional image shown in Fig. 2 or 3 while maintaining the existing Y-axis information or partly maintaining the existing horizontal plane information, the three-dimensional image can be displayed in any of various orientations based on the landmark with improved reproducibility. Therefore, this method is very useful for the morphological evaluation and the measurement of the facial bones.

In this embodiment, as will be described below, new reference axes are defined by finely adjusting the X-axis, the Y-axis and the Z-axis defined based on a landmark in the displayed image shown in Fig. 2 while partly maintaining information of the X-axis, the Y-axis and the Z-axis.
Further, new reference planes are defined by finely adjusting the horizontal reference plane HP based on the position of a landmark not present on the existing horizontal reference plane HP while partly maintaining information of the existing horizontal reference plane HP.

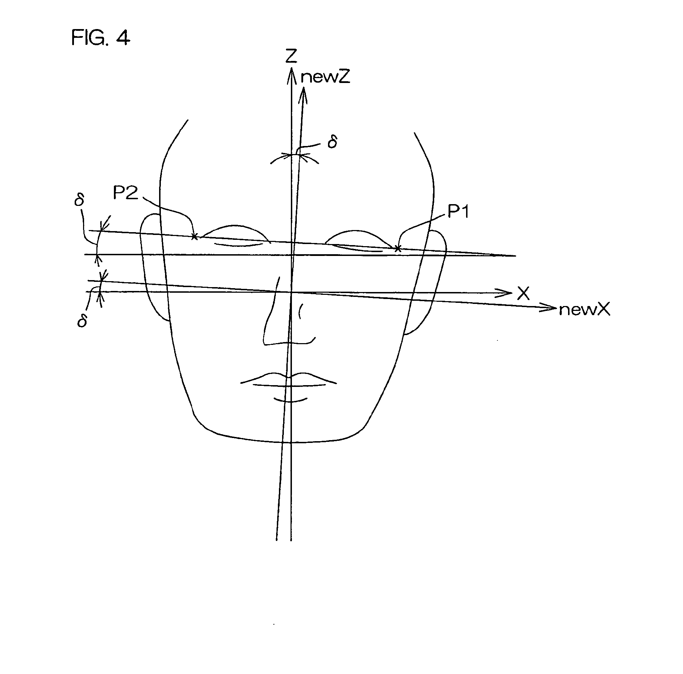

Fig. 4 is a diagram for explaining a method for modifying the X-axis in the three-dimensional image of the human head displayed based on the landmark-based reference axes (the X-axis, the Y-axis and the Z-axis) shown in Fig. 2.
It is herein assumed that the three-dimensional image of the human head is a 3D-CT image (three-dimensional CT image). By adjusting CT values in the CT image, cranial bones as an internal structure or a skin as an exterior geometry can be displayed. For example, the skin is herein displayed by adjusting the CT values. Then, a right outer canthus P1 and a left outer canthus P2 are specified by a cursor, for example, by operating the mouse 4.

In response to this, the control section 1 registers the specified points P1, P2 as new landmarks, and calculates an angle δ defined between the X-axis and a line extending through the points P1, P2. That is, the control section 1 calculates the inclination of the line extending through the points P1. P2 with respect to the X-axis.
Then, the X-axis is inclined at the angle δ about the Y-axis so as to be parallel to the line extending through the points P1, P2. The resulting X-axis is defined as a new X-axis.

As the X-axis is inclined at the angle δ, the Z-axis is also inclined at the angle δ. The resulting Z-axis is defined as a new Z-axis.
As a result, the new X-axis, the original Y-axis and the new Z-axis are used as new reference axes for displaying the three-dimensional image. Thus, the X-axis and the Z-axis are modified with the positional coordinate on the original Y-axis kept unchanged.
The new reference axes, i.e., the new X-axis, the original Y-axis and the new Z-axis, are defined based on the original landmarks as well as the new landmarks P1, P2 and, therefore, are anatomically reproducible.

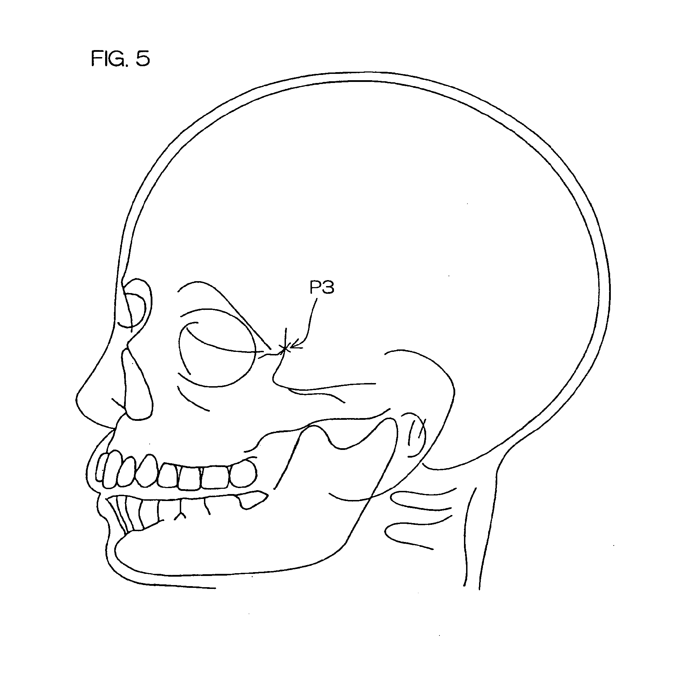

Next, a method for modifying the display reference planes in the three-dimensional image of the human head displayed based on the horizontal reference plane HP, the frontal plane VP1 and the median plane VP2 shown in Fig. 3 will be described.
The three-dimensional image of the human head displayed based on the horizontal reference plane HP, the frontal plane VP1 and the median plane VP2 is rotated into a desired orientation, and the resulting image is displayed as viewed from a left side of the head as shown in Fig. 5 by adjusting the CT values.

A frontozygomatic suture point (outer side) P3 of the cranial bones is specified in the image, and registered as a landmark. The registration may be achieved by pointing the frontozygomatic suture point (outer side) by the cursor and clicking the mouse 4.
Subsequently, though not shown, the displayed human head image is rotated. Thus, the human head image is displayed as viewed from a right side of the human head. Then, a right frontozygomatic suture point (outer side) P4 is registered as a landmark.

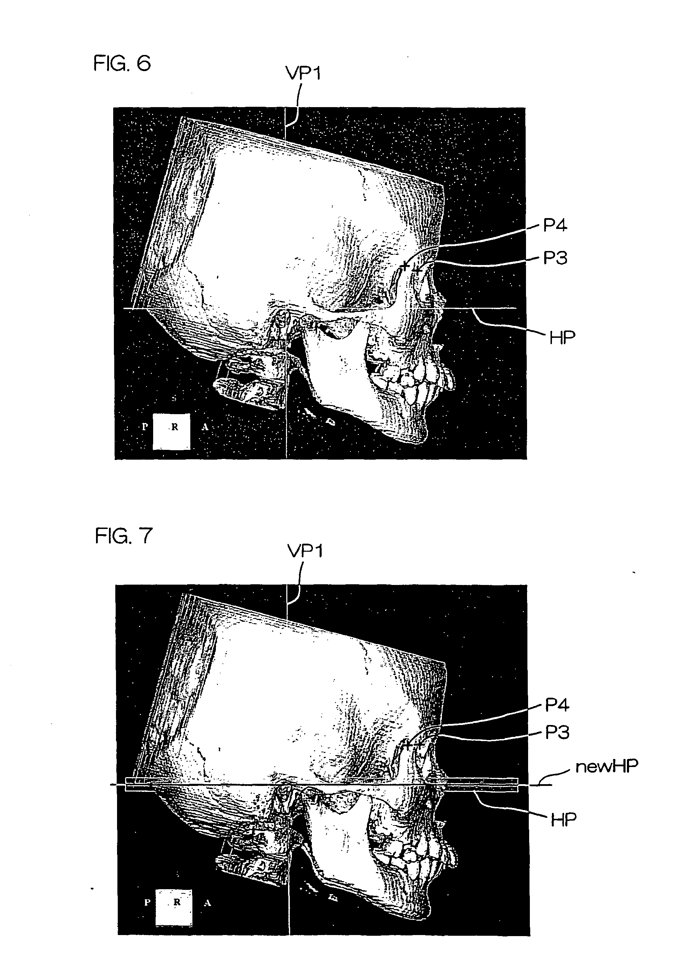

In turn, the three-dimensional image of the human head is displayed as viewed perpendicularly to the median plane VP2 from the right side of the human head as shown in Fig. 6. At this time, the landmarks P3, P4 are also displayed.
Then, the displayed three-dimensional image is automatically or manually rotated about the frontal plane VP1 so that the two landmarks P3 and P4 are horizontally aligned. A three-dimensional image resulting from the rotation is shown in Fig. 7. In the three-dimensional image of Fig. 7, the orientation of the frontal plane VP1 is kept unchanged, and the horizontal reference plane HP is rotated about the Y-axis by a predetermined angle (an angle such as to horizontally align the landmarks P3 and P4).

In turn, the horizontal reference plane HP is modified so that the orientation of the image shown in Fig. 7 corresponds to a new right side view. Thus, the modified horizontal reference plane is defined as a new horizontal plane.
In this embodiment, the frontal plane VP1 is kept unchanged, and the orientation of the horizontal reference plane HP is modified. Correspondingly, the median plane VP2 is also rotated to be modified so as to be perpendicular to the modified horizontal reference plane HP.

Then, the modified horizontal reference plane _{new}HP, the original frontal plane VP1 and the modified median plane _{new}VP2 are defined as new reference planes, and the three-dimensional image is displayed in an orientation defined based on the reference planes.
The image displayed based on the reference planes defined based on the landmarks is modified based on the reference planes modified based on the additional landmarks defined at the right and left frontozygomatic suture points, and the resulting image is displayed as a new image. Therefore, the new image is displayed based on the anatomically reproducible reference planes.

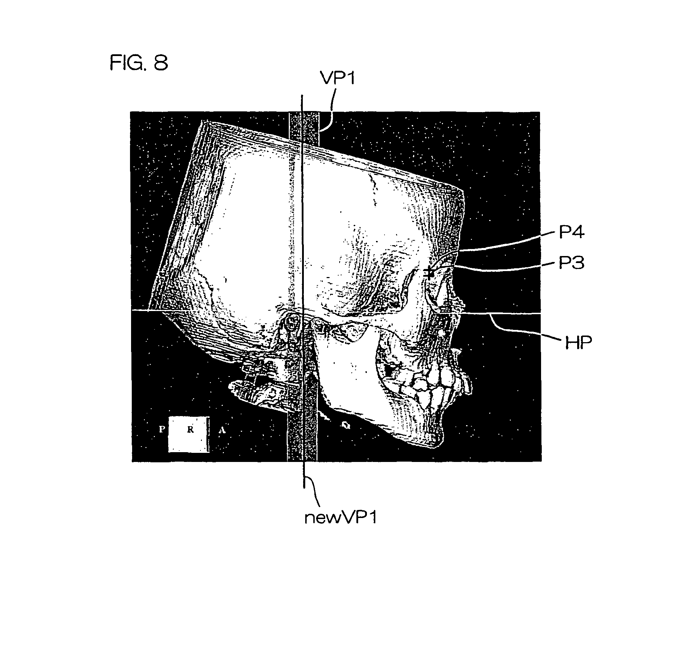

In the display image shown in Fig. 6, the landmarks P3, P4 may be caused to match each other in lateral position as shown in Fig. 8 rather than in height. With this arrangement, the reference planes for the display orientation of the three-dimensional image are rotated about the horizontal reference plane HP. Thus, the frontal plane VP1 is modified.

As shown in Fig. 8, the frontal plane VP1 is rotated from the original frontal plane VP1 by a minute angle (about the Z-axis). The resulting frontal plane VP1 is defined as a new frontal plane _{new}VP1. That is, in Fig. 8, the new frontal plane _{new}VP1 is defined by a widthwise center line of the frontal plane VP1 which extends vertically. In this case, the median plane VP2 perpendicular to the frontal plane VP1 is also automatically modified.

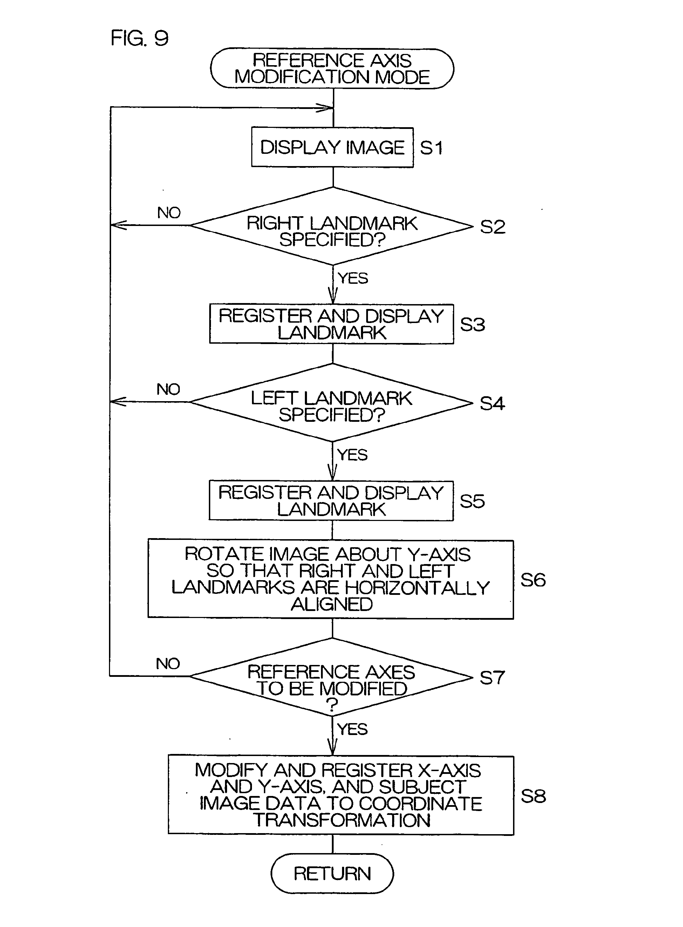

Fig. 9 is a flow chart showing a process sequence to be performed by the control section 1 for modifying the reference axes.
Next, a reference axis modifying operation to be performed by the control section 1 will be described with reference to Fig. 9.
The control section 1 is permitted to perform the reference axis modifying operation when a specific mode, e.g., a reference axis modification mode, is selected.

In the reference axis modification mode, a medical three-dimensional image is displayed on the display device 2 (Step S1). Then, a user rotates the displayed three-dimensional image in a desired direction by a desired angle or adjusts the CT values so that a right landmark to be specified by the user appears in the three-dimensional image. Then, the user specifies the displayed right landmark, for example, by the mouse 4 or the like.
In response to the user specifying the right landmark, the control section 1 registers the specified landmark, which is in turn displayed in superposition on the three-dimensional image (Step S3).

Further, the user rotates the three-dimensional image displayed on the display device 2 so that a left landmark appears in the three-dimensional image, and specifies the left landmark by the mouse 4 or the like. In response to the user specifying the left landmark (YES in Step S4), the control section 1 registers the left landmark, which is in turn displayed in superposition on the three-dimensional image on the display device 2 (Step S5).
Then, the control section 1 rotates the three-dimensional image about the Y-axis so that the right and left landmarks are aligned horizontally (Step S6). This rotating operation may be automatically performed by the control section 1 or manually performed by the user.

With the right and left landmarks aligned horizontally, the user inputs a command for the reference axis modifying operation (or inputs a modification signal by means of the mouse 4 or the keyboard 3). In response to the input (YES in Step S7), the control section 1 modifies the X-axis and the Z-axis, and registers a new X-axis and a new Z-axis.
Then, three-dimensional image data is subjected to coordinate transformation based on the modified X- and Z-axes and the original Y-axis (which is kept unchanged) (Step S8).

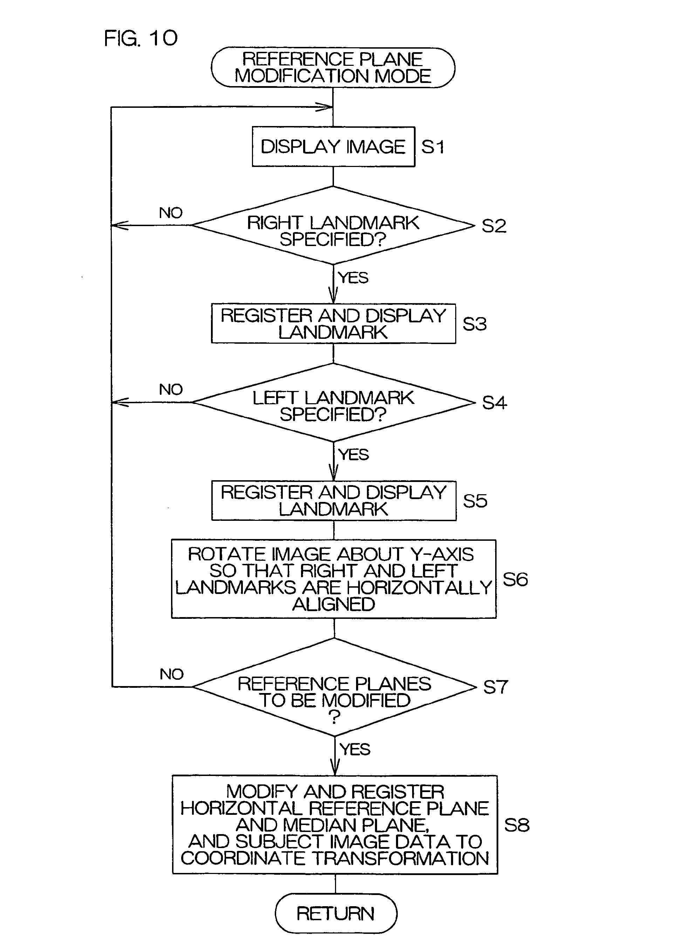

Fig. 10 is a flow chart showing a process sequence to be performed by the control section 1 for modifying the reference planes.
Next, a reference plane modifying operation to be performed by the control section 1 will be described with reference to Fig. 10.
The control section 1 is permitted to perform the reference plane modifying operation when a specific mode, e.g., a reference plane modification mode, is selected.

In the reference plane modification mode, a medical three-dimensional image is displayed on the display device 2 (Step S1). Then, a user rotates the displayed three-dimensional image in a desired direction by a desired angle or adjusts the CT values so that a right landmark to be specified by the user appears in the three-dimensional image. Then, the user specifies the displayed right landmark, for example, by the mouse 4 or the like.
In response to the user specifying the right landmark, the control section 1 registers the specified landmark, which is in turn displayed in superposition on the three-dimensional image (Step S3).

Further, the user rotates the three-dimensional image displayed on the display device 2 so that a left landmark appears in the three-dimensional image, and specifies the left landmark by the mouse 4 or the like. In response to the user specifying the left landmark (YES in Step S4), the control section 1 registers the left landmark, which is in turn displayed in superposition on the three-dimensional image on the display device 2 (Step S5).
Then, the control section 1 rotates the three-dimensional image about the frontal plane so that the right and left landmarks are aligned horizontally (Step S6). This rotating operation may be automatically performed by the control section 1 or manually performed by the user.

With the right and left landmarks aligned horizontally, the user inputs a command for the reference plane modifying operation (or inputs a modification signal by means of the mouse 4 or the keyboard 3). In response to the input (YES in Step S7), the control section 1 modifies the horizontal reference plane and the median plane, and registers a new horizontal reference plane and a new median plane.
Then, three-dimensional image data is subjected to coordinate transformation based on the horizontal reference plane and the median plane which are newly registered and the original frontal plane (which is kept unchanged).

Description has thus been given to how to modify the X-axis and the Z-axis based on the image shown in Fig. 2, and how to modify the horizontal reference plane HP and the median plane VP2 based on the image shown in Fig. 3.
Further, description has been given to the modification of the frontal plane VP1 and the median plane VP2 with the horizontal reference plane HP kept unchanged in the image shown in Fig. 3 (description given with reference to Fig. 8).
As described above, one of the X-axis, the Y-axis and the Z-axis defined as the reference axes based on the landmarks in the medical three-dimensional image is kept unchanged, and the other two reference axes are modified. Where the medical three-dimensional image is displayed based on the three orthogonal reference planes, one of the three reference planes is kept unchanged, and the other two reference planes are modified.

With these arrangements, the orientation of the medical three-dimensional image can be modified as desired by utilizing the landmarks. Therefore, the medical three-dimensional image can be displayed in any of various orientations with improved reproducibility. As a result, an image of facial bones to be evaluated or measured can be displayed in any desired orientation with improved reproducibility, for example, for morphological evaluation of the facial bones.
It should be understood that the present invention be not limited by the foregoing description. The present invention is applicable to the modification of the following coordinate systems including reference axes or reference planes.

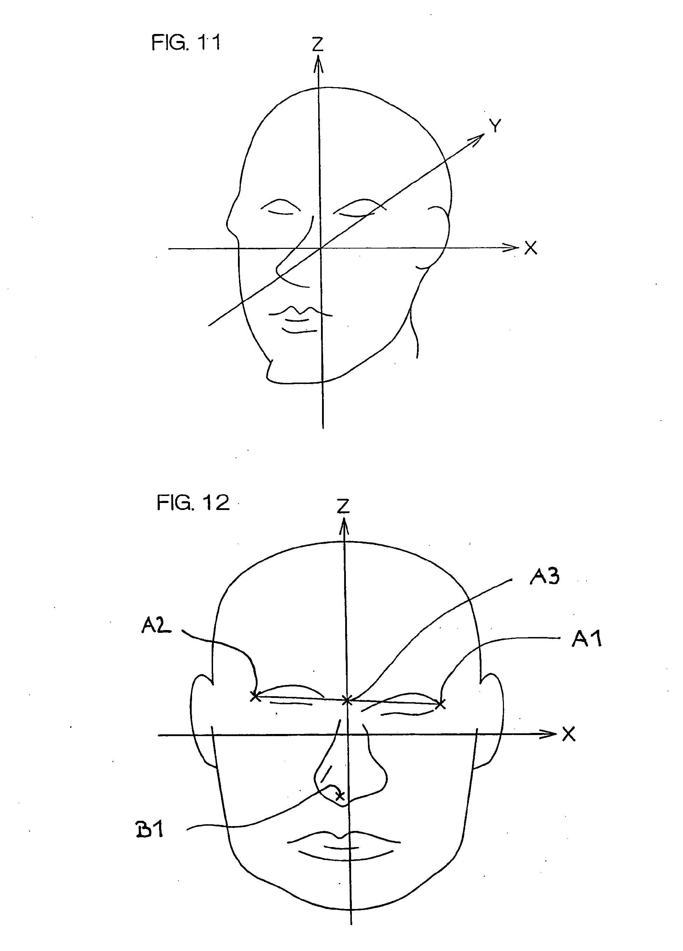

Fig. 11 illustrates a three-dimensional image of a human head displayed based on an X-axis, a Y-axis and a Z-axis of an existing reference coordinate system defined based on landmarks, and Fig. 12 is a diagram of the three-dimensional image of the human head displayed as viewed from a front side with the X-axis and the Z-axis indicated by straight lines. The Y-axis anteroposteriorly extends through an origin defined by an intersection of the X-axis and the Z-axis, and is illustrated as a point.
Next, how to modify the X-axis and the Z-axis to modify the display orientation of the three-dimensional image will be described.

A right outer canthus A1 and a left outer canthus A2 appearing in a front view of the human head in Fig. 12 are specified, for example, by the mouse 4 or the like (see Fig. 1). In response to the specification of the right and left outer canthuses A1, A2, the control section 1 automatically calculates a middle point of a line segment A1-A2, i.e., a middle point A3 between the right and left outer canthuses. Then, the control section 1 registers the calculated middle point as a landmark, and displays the landmark on the image of the human head.

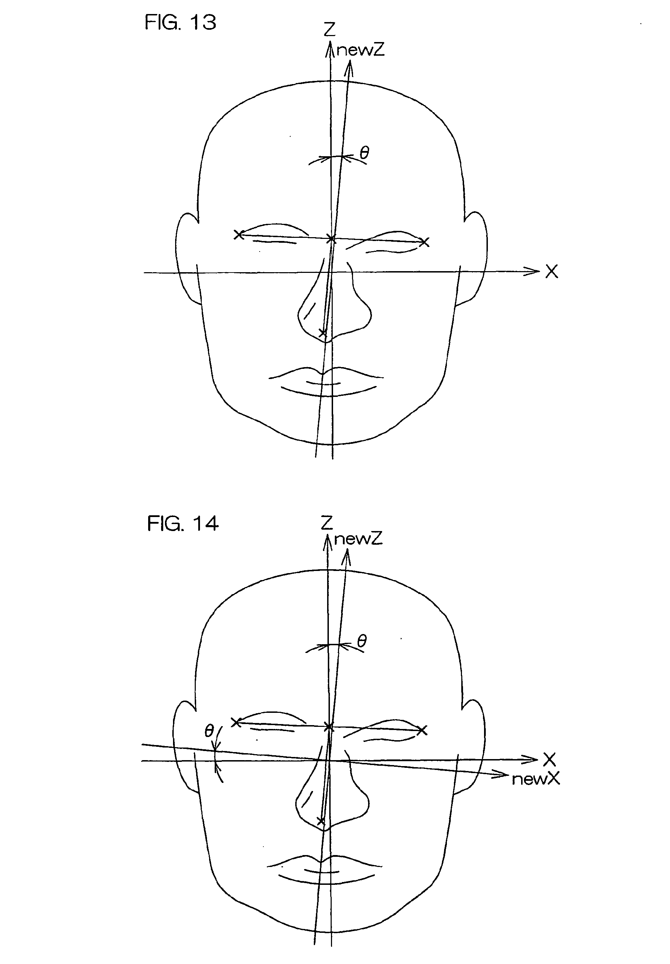

Further, the user specifies a nose tip B1 by the mouse 4 or the like. In response to this, the position of the nose tip B1 is registered as a landmark, which is in turn displayed on the image of the human head. Then, as shown in Fig. 13, the Z-axis is rotated about the Y-axis so as to be parallel to a line segment A3-B1, and the resulting Z-axis is defined as a new Z-axis.
Then, as shown in Fig. 14, the X-axis orthogonal to the Z-axis is also rotated about the Y-axis by the same angle ⊝ as the Z-axis by the definition of the new Z-axis, and the resulting X-axis is defined as a new X-axis.

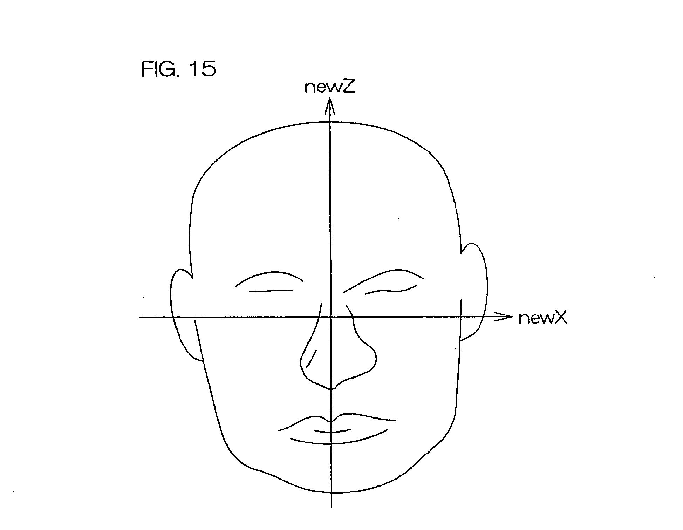

As a result, the existing Y-axis is used as the Y-axis, and the modified X- and Z-axes are used as the new axes. Then, a front view of the three-dimensional image of the human head is displayed based on a new reference coordinate system, i.e., the new X-axis, the Y-axis and the new Z-axis, as shown in Fig. 15.
While the coordinate system defined by the X-axis, the Y-axis and the Z-axis is used as the reference coordinate system based on the landmarks by way of example, the coordinate system defined by the horizontal reference plane, the frontal plane and the median plane may be used as the reference coordinate system based on the landmarks for displaying the three-dimensional image. Even in this case, the reference coordinate system can be modified in a similar manner by rotating the median plane and the horizontal reference plane with the frontal plane kept unchanged.

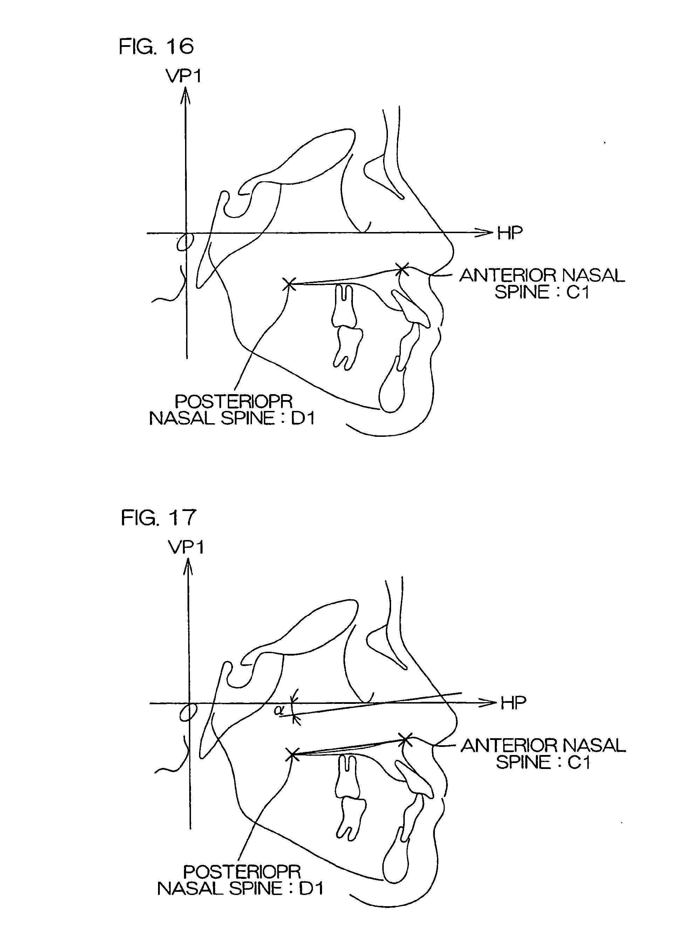

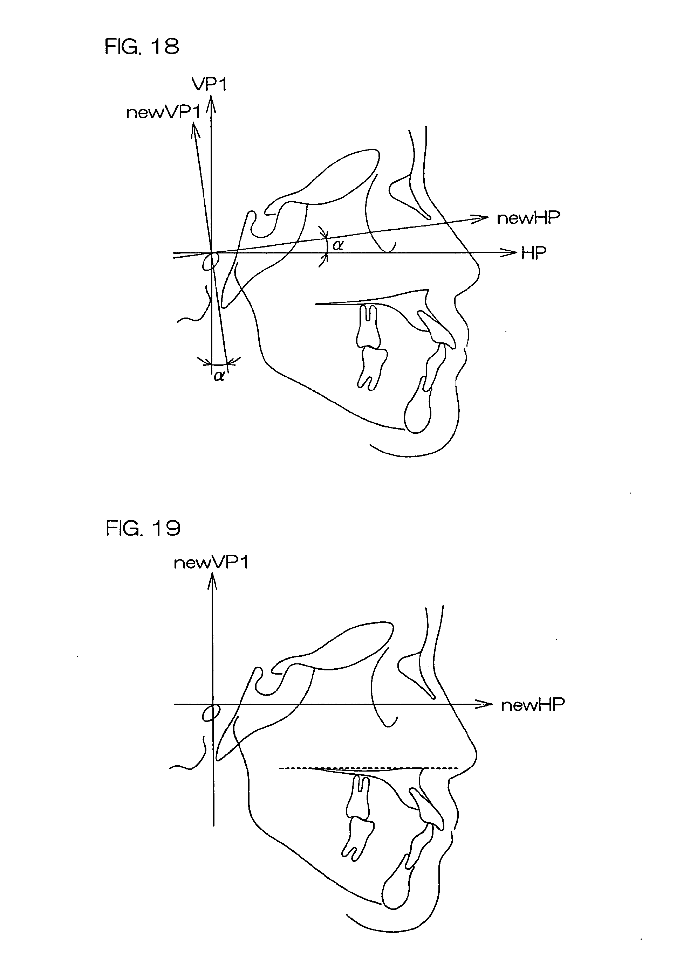

Figs. 16 to 19 are diagrams for explaining another exemplary method for modifying the reference coordinate system.
Figs. 16 to 19 illustrate a lateral front portion of a human head displayed by a three-dimensional Raysum display method (isotropic equivalent projection display method). In addition to the human head, a horizontal reference plane HP and a frontal plane VP1 are displayed as lines in Fig. 16. In Fig. 16, an anterior nasal spine C1 and a posterior nasal spine D1 are specified by the mouse or the like. In response to the specification, the points C1, D1 are registered as landmarks. Further, an angle α defined between a line segment C1-D1 and the horizontal reference plane HP is calculated. An exemplary method for the calculation includes the steps of projecting the two points C1 D1 onto the median plane, translating the line segment C1-D1 on the median plane and determining the angle α defined between the line segment C1-D1 and the horizontal reference plane HP (see Fig. 17).
Next, as shown in Fig. 18, the horizontal reference plane HP and the frontal plane VP1 are rotated about the X-axis by an angle α, whereby a new horizontal reference plane _{new}HP and a new frontal plane _{new}VP1 are defined. The original median plane is used as it is.

As a result, a side view of the three-dimensional image is displayed based on a coordinate system defined by the new reference planes resulting from the modification as shown in Fig. 19. In Fig. 19, the heights of the anterior nasal spine and the posterior nasal spine are defined with respect to the new horizontal reference plane _{new}HP or, in other words, a phantom line connecting the anterior nasal spine and the posterior nasal spine is defined, and the phantom line is displayed as extending parallel to the new horizontal reference plane _{new}HP.
Further, the present invention is applicable to the following coordinate system modification methods for modifying the reference axes and the reference planes.

In figures preceding Fig. 10, as described above, a pair of landmarks laterally aligned on the human head are specified as the landmarks for the modification of the reference coordinate system by way of example. In figures following Fig. 11, the modification of the reference coordinate system based on a pair of landmarks vertically aligned on the human head, and the modification of the reference coordinate system based on a pair of landmarks anteroposteriorly aligned are shown by way of examples.
As apparent from these examples, the landmarks for the modification of the reference coordinate system may be any pair of landmarks in the image, and the landmark aligning direction is not limited to the lateral direction, the vertical direction and the anteroposterior direction.

For example, the coordinate system and the display orientation of the three-dimensional image may be modified by adjusting the CT values to display a skin on a 3D-CT image positioned with respect to an oculoauricular plane, specifying right and left outer canthuses, projecting the positional coordinates of these two points on the frontal plane, and inclining the X-axis and the Z-axis at an angle defined between the horizontal reference plane (X-axis) and a line connecting the right and left outer canthuses projected on the frontal plane.
Similarly, the X-axis and the Z-axis may be modified by specifying two points of right and left outer canthuses, determining a middle point between the two points, specifying a nose tip, projecting the middle point and the nose tip on the frontal plane, and inclining the X-axis and the Z-axis at an angle defined between the Z-axis and a line connecting the two points projected on the frontal plane.

Similarly, the Y-axis and the Z-axis may be modified by specifying an anterior nasal spine and a posterior nasal spine which are located at an anterior end and a posterior end of a maxillary bone, projecting these two points on the median plane, and rotating the Y-axis and the Z-axis by an angle defined between the Z-axis and a line connecting the two points projected on the frontal plane.
Further, the reference axes may be modified by specifying a plurality of pairs of laterally aligned characteristic points such as right and left inner canthuses, right and left nose wings and right and left mouth corners on 3D image of a skin located with respect to an oculoauricular plane, projecting these points on the frontal plane, calculating an average of angles between the X-axis and lines each connecting the associated projected points, and inclining the X-axis and the Z-axis at the calculated average angle.

In any of the aforementioned cases, a plurality of characteristic points, rather than a single characteristic point, may be specified on each of the right and left sides, and a middle or a center of the points may be specified on each of the right and left sides.
It should be understood that the present invention be not limited to the embodiments described above, but various modifications may be made within the scope of the present invention defined by the appended claims.

## Claims

1. A medical three-dimensional image display orientation modifying apparatus, comprising:
displaying means (2) that displays a medical three-dimensional image based on a three-dimensional coordinate system defined based on an anatomical characteristic point referred to as reference landmark contained in the image;
**characterised in that** it further comprises registering means that, when a pair of right and left landmarks (P₁, P₂; P₃, P₄; A₃, B₁; C₁, D₁) different from the reference landmark used for the definition of the original three-dimensional coordinate system are specified in right and left regions of the displayed medical three-dimensional image, registers the specified landmarks; and
modifying means that modifies inclinations of two (X, Z; HP, VP₁) of three axes of the three-dimensional coordinate system based on the pair of registered right and left landmarks (P₁, P₂; P₃, P₄; A₃, B₁; C₁, D₁) with the other axis (Y; VP₂) kept unchanged.

2. A medical three-dimensional image display orientation modifying apparatus as set forth in claim 1,
wherein the original medical three-dimensional image includes an image of a human head displayed based on a horizontal reference plane, a frontal plane and a median plane defined based on the reference landmark,
wherein the modifying means modifies inclinations of the horizontal reference plane and the median plane based on the pair of registered right and left landmarks with the frontal plane kept unchanged.

3. A medical three-dimensional image display orientation modifying apparatus as set forth in claim 1,
wherein the original medical three-dimensional image includes an image of a human head displayed based on a horizontal reference plane, a frontal plane and a median plane defined based on the reference landmark,
wherein the modifying means modifies inclinations of the frontal plane and the median plane based on the pair of registered right and left landmarks with the horizontal reference plane kept unchanged.

4. A medical three-dimensional image display orientation modifying apparatus as set forth in any of claims 1 to 3,
wherein a plurality of landmarks are specified for each of the right and left landmarks to be registered, and the right and left landmarks are each defined by a middle point of the plurality of landmarks.

5. A medical three-dimensional image display orientation modifying method, comprising the steps of:
displaying a medical three-dimensional image based on a three-dimensional coordinate system defined based on a reference landmark contained in the image;
**characterised in**, when a pair of landmarks different from the reference landmark used for the definition of the original three-dimensional coordinate system are specified in the displayed medical three-dimensional image for modification of the coordinate system, registering the specified landmarks; and
modifying inclinations of two of three orthogonal planes of the three-dimensional coordinate system based on the pair of registered landmarks with the other plane kept unchanged.

6. A medical three-dimensional image display orientation modifying program which causes an apparatus as recited in any of claims 1 to 4 to perform a medical three-dimensional image display orientation modifying process.

## Patentansprüche

1. Vorrichtung zur Modifizierung der Anzeigeorientierung eines medizinischen dreidimensionalen Bildes, mit:
Anzeigemitteln (2), die ein medizinisches dreidimensionales Bild auf der Grundlage eines dreidimensionalen Koordinatensystems anzeigen, das auf der Grundlage eines anatomischen charakteristischen Punktes definiert ist, der als Referenzlandmarke bezeichnet wird und in dem Bild enthalten ist;
**dadurch gekennzeichnet, dass** die Vorrichtung ferner aufweist:
Erfassungsmittel, die dann, wenn ein Paar einer rechten und einer linken Landmarke (P₁, P₂; P₃, P₄; A₃, B₁; C₁, D₁), die sich von der Referenzlandmarke unterscheiden, die für die Definition des ursprünglichen dreidimensionalen Koordinatensystems verwendet wird, in einer rechten und einer linken Region des angezeigten medizinischen dreidimensionalen Bildes spezifiziert sind, diese spezifizierten Landmarken erfassen; und
Modifizierungsmittel, die die Neigungen von zwei (X, Z; HP, VP₁) von drei Achsen des dreidimensionalen Koordinatensystems modifizieren, und zwar auf der Grundlage des Paars von erfasster rechter und linker Landmarke (P₁, P₂; P₃, P₄; A₃, B₁; C₁, D₁), wobei die andere Achse (Y; VP₂) unverändert belassen wird.

2. Vorrichtung zur Modifizierung der Anzeigeorientierung eines medizinischen dreidimensionalen Bildes nach Anspruch 1,
wobei das ursprüngliche medizinische dreidimensionale Bild ein Bild eines menschlichen Kopfes aufweist, der auf der Grundlage einer horizontalen Referenzebene, einer Frontalebene und einer Medianebene angezeigt wird, die auf der Grundlage der Referenzlandmarke definiert sind,
wobei die Modifizierungsmittel die Neigungen der horizontalen Referenzebene und der Medianebene modifizieren, und zwar auf der Grundlage des Paars der erfassten rechten und linken Landmarke, wobei die Frontalebene unverändert belassen wird.

3. Vorrichtung zur Modifizierung der Anzeigeorientierung eines medizinischen dreidimensionalen Bildes nach Anspruch 1,
wobei das ursprüngliche medizinische dreidimensionale Bild ein Bild eines menschlichen Kopfes aufweist, der auf der Grundlage einer horizontalen Referenzebene, einer Frontalebene und einer Medianebene angezeigt wird, die auf der Grundlage der Referenzlandmarke definiert sind,
wobei die Modifizierungsmittel die Neigungen der Frontalebene und der Medianebene modifizieren, und zwar auf der Grundlage des Paars der erfassten rechten und linken Landmarke, wobei die horizontale Referenzebene unverändert belassen bleibt.

4. Vorrichtung zur Modifizierung der Anzeigeorientierung eines medizinischen dreidimensionalen Bildes nach einem der Ansprüche 1 bis 3,
wobei eine Vielzahl von Landmarken für jede der rechten und der linken zu erfassenden Landmarke spezifiziert sind, und wobei die rechte und die linke Landmarke jeweils durch einen Mittelpunkt der Vielzahl von Landmarken definiert sind.

5. Verfahren zur Modifizierung der Anzeigeorientierung eines medizinischen dreidimensionalen Bildes, mit den Schritten:
Anzeigen eines medizinischen dreidimensionalen Bildes auf der Grundlage eines dreidimensionalen Koordinatensystems, das auf der Grundlage einer Referenzlandmarke definiert ist, die in dem Bild enthalten ist,
**gekennzeichnet durch**
den Schritt, dann, wenn ein Paar von Landmarken, die sich von der Referenzlandmarke unterscheiden, die für die Definition des ursprünglichen dreidimensionalen Koordinatensystems verwendet wird, in dem angezeigten medizinischen dreidiModifizieren von Neigungen von zwei von drei orthogonalen Ebenen des dreidimensionalen Koordinatensystems auf der Grundlage des Paares von erfassten Landmarken, wobei die andere Ebene unverändert belassen wird.

6. Programm zur Modifizierung der Anzeigeorientierung eines medizinischen dreidimensionalen Bildes, wobei das Programm veranlasst, dass eine Vorrichtung nach einem der Ansprüche 1 bis 4 einen Prozess zur Modifizierung der Anzeigeorientierung eines medizinischen dreidimensionalen Bildes durchführt.

## Revendications

1. Appareil de modification de l'orientation d'affichage d'images tridimensionnelles, comprenant :
un moyen d'affichage (2) qui affiche une image tridimensionnelle médicale par rapport à un système de coordonnées tridimensionnel défini sur la base d'un point anatomique caractéristique appelé point de repère de référence contenu dans l'image ;
**caractérisé en ce qu'**il comprend en outre un moyen d'alignement qui, lorsqu'une paire de points de repère droit et gauche (P₁, P_{2;} P₃, P_{4;} A₃, B₁ ; C₁, D₁) différents du point de repère de référence utilisé pour la définition du système de coordonnées tridimensionnel d'origine est spécifiée dans des régions droite et gauche de l'image tridimensionnelle médicale affichée, aligne les points de repère spécifiés ; et
un moyen de modification qui modifie les inclinaisons de deux (X, Z ; HP, VP₁) de trois axes du système de coordonnées tridimensionnel sur la base de la paire des points de repère droit et gauche alignés (P₁, P_{2;} P₃, P_{4;} A₃, B₁ ; C₁, D₁), l'autre axe (Y ; VP₂) étant maintenu inchangé.

2. Appareil de modification de l'orientation d'affichage d'une image médicale tridimensionnelle selon la revendication 1,
dans lequel l'image médicale tridimensionnelle d'origine comprend une image d'une tête humaine affichée par rapport à un plan de référence horizontal, un plan frontal et un plan médian définis sur la base du point de repère de référence,
dans lequel le moyen de modification modifie des inclinaisons du plan de référence horizontal et du plan médian par rapport à la paire de points de repère droit et gauche alignés, le plan frontal étant maintenu inchangé.

3. Appareil de modification de l'orientation d'affichage d'une image médicale tridimensionnelle selon la revendication 1,
dans lequel l'image médicale tridimensionnelle d'origine comprend une image d'une tête humaine affichée par rapport à un plan de référence horizontal, à un plan frontal et à un plan médian définis sur la base du point de repère de référence,
dans lequel le moyen de modification modifie des inclinaisons du plan frontal et du plan médian par rapport à la paire de points de repère droit et gauche alignés, le plan de référence horizontal étant maintenu inchangé.

4. Appareil de modification de l'orientation d'affichage d'une image médicale tridimensionnelle selon l'une quelconque des revendications 1 à 3,
dans lequel une pluralité de points de repère sont spécifiés pour chacun des points de repère droit et gauche devant être alignés, et les points de repère droit et gauche sont chacun définis par un point milieu de la pluralité de points de repère.

5. Procédé de modification de l'orientation d'affichage d'une image médicale tridimensionnelle, comprenant les étapes consistant à :
afficher une image médicale tridimensionnelle dans un système de coordonnées tridimensionnel défini par rapport à un point de repère de référence contenu dans l'image ;
**caractérisé**, lorsqu'une paire de points de repère différents du point de repère de référence utilisé pour la définition du référentiel tridimensionnel d'origine est spécifiée dans l'image médicale tridimensionnelle affichée pour la modification du référentiel, par le fait d'aligner les points de repère spécifiés ; et
modifier les inclinaisons de deux de trois plans orthogonaux du système de coordonnées tridimensionnel par rapport à la paire de points de repère alignés, l'autre plan étant maintenu inchangé.

6. Programme de modification de l'orientation d'affichage d'une image médicale tridimensionnelle qui fait en sorte qu'un appareil selon l'une quelconque des revendications 1 à 4 effectue un processus de modification de l'orientation d'affichage d'une image médicale tridimensionnelle.
